# EUROPÄISCHE PATENTSCHRIFT

(11) **EP 1 993 667 B1**
(45) Veröffentlichungstag und Bekanntmachung des Hinweises auf die Patenterteilung: **22.06.2011**
(21) Anmeldenummer: 06723469.0
(22) Anmeldetag: 16.03.2006
(51) Int. Cl.: A61N 2/02

(54) **PORTABLER APPLIKATOR ZUR KOLLAGENSTIMULATION**
PORTABLE APPLICATOR FOR COLLAGEN STIMULATION
APPLICATEUR PORTABLE POUR LA STIMULATION AU COLLAGÈNE

(43) Veröffentlichungstag der Anmeldung: 26.11.2008
(73) Patentinhaber: Binder Markoll, Ernestine, 07157 Andratx (ES)
(72) Erfinder: MARKOLL, Richard, 07157 Andratx (ES); BINDER MARKOLL, Ernestine, 07157 Andratx (ES)
(74) Vertreter: Zimmermann & Partner
(86) Internationale Anmeldenummer: PCT/EP2006/002421
(87) Internationale Veröffentlichungsnummer: WO 2007/104340

(56) Entgegenhaltungen:
- EP-A2- 0 244 784
- US-A- 5 691 325
- US-B1- 6 463 336

## Beschreibung

### Gebiet der Erfindung

Die Erfindung betrifft einen portablen Applikator zum Einsatz zur pulsierenden Signal-Therapie zur Behandlung von Hautveränderungen, beispielsweise bedingt durch Hautalterung oder Brandverletzungen, und insbesondere einen portablen Applikator zum Einsatz zur pulsierenden Signal-Therapie zur Stimulation der Kollagenneubildung.

Dabei ist es wesentlich, dass die pulsierenden Magnetfelder auf die entsprechenden Körperstellen in geeigneter Stärke und mit einer geeigneten Frequenz über eine bestimmte Zeitdauer aufgebracht werden, so dass Kollagenenthaltende Gewebesysteme des Körpers angeregt werden.

### Hintergrund der Erfindung

Die menschliche Haut besteht aus mehreren Schichten, der Oberhaut (Epidermis), der Lederhaut (Dermis) und der Unterhaut (Subcutis). Begrenzt wird die Haut durch die allgemeine Körperfaszie, die aus Kollagenfasern besteht. Nach der Faszie folgt je nach Körperregion entweder Muskulatur, Knochen, Knorpel oder Fett. Die Lederhaut ist eine elastische Hautschicht, die zu einem großen Teil aus den Strukturproteinen Kollagen und Elastin zusammengesetzt ist. So enthält sie u.a. ein dichtes Netz aus Kollagenfasern, das mit elastischem Bindegewebe gefüllt ist. Knorpel ist ein Stützgewebe, das aus wasserreichen Knorpelzellen (Chondrozyten) und Interzellularsubstanz besteht. Die extrazelluläre Matrix des Knorpels besteht zu 60 bis 80 % aus Wasser. Die Interzellularsubstanz setzt sich aus Grundsubstanz (Proteoglykanen und Glukoproteinen) sowie Fasern (Kollagenen) zusammen. Proteoglykane, Glukoproteine und Kollagene werden von Chondrozyten hergestellt. Die Kollagene bilden ein Geflecht, worin Proteoglykane und Glukoproteine eingelagert und fixiert sind.

Es gibt viele verschiedene Arten von Kollagenen. Alle Kollagene haben gemein, dass sie aus drei Polypeptidketten aufgebaut sind, die in Form einer Tripelhelix umeinander gewunden sind. Kollagenmoleküle des Typs I, II, III, V und VI, die als "fibrilläre Kollagene" bezeichnet werden, haben ähnliche Molekularstrukturen. Kollagenmoleküle des Typs IX, XII und XIV, sogenannte "fibrillenassoziierte Kollagene", enthalten mehrere Tripelhelix-Domänen, die durch nichtkollagene Domänen unterbrochen sind. Innerhalb der verschiedenen Typen von Kollagenmolekülen lassen sich auch signifikante Unterschiede feststellen. Kollagenmoleküle des Typs V haben einen niedrigen Alaningehalt, aber einen hohen Gehalt an basischen Aminosäuren. Außerdem schließen sie sich zu Tetrameren zusammen. Der Typ VIII ist eine starke Protease. Der Typ IX ist auf der Oberfläche von Kollagen Typ II zu finden. Kollagenmoleküle des Typs XI sind kurze Tripelhelices mit sehr langen globulären Ausdehnungen. Kollagenmoleküle des Typs VII haben einen N-terminalen Domänenbereich mit drei Fingern, ähnlich den Kollagentypen XII und XIV.

Mit zunehmendem Alter verändert sich der Aufbau der verschiedenen Hautschichten: Die jeweiligen Hautschichten werden dünner, die Fettschichten nehmen ab, und die Strukturproteine der Lederhaut zerfallen, was sich wiederum als Falten auswirkt.

Im Stand der Technik sind Verfahren zur Behandlung von faltiger oder alternder Haut, bzw. von Hautverfärbungen, mittels einer Magnetfeldtherapie bekannt. Ein derartiges Verfahren wird beispielsweise in dem U.S. Patent Nr. 5,669,868, den Erfinder R. Markoll benennend, beschrieben. Bei diesem Verfahren wird die Haut einem Magnetfeld, erzeugt durch einen Gleichstrom mit einer rechteckigen Wellenform, von weniger als 20 Gauß ausgesetzt. Durch das angelegte Magnetfeld wird die Bildung von Kollagen angeregt. In dem U.S. Patent Nr. 5,669,868 wird das Magnetfeld durch eine ringförmige Spule erzeugt, innerhalb derer der jeweilige Körperteil zur Behandlung positioniert wird.

Das Dokument US 6,463,336 offenbart eine Hochfrequenz- oder Mikrowellen-Bandage für die therapeutische Behandlung. Dabei entsteht das Magnetfeld durch "antennas", die planar zur Oberflächenstruktur liegen.

Dokument EP-A2-0244784 beschreibt ein elektrotherapeutisches Magnetfeld-Heilgerät zu Erzielung einer Heilwirkung während alltäglicher Tätigkeiten eines Patienten. Das Gerät enthält eine Anzahl von Spulen, die beispielsweise in einen Autositz eingebaut werden. Die Spulen sind parallel zur Oberflächenstruktur orientiert. Über die Schaltung der Spulen enthält das Dokument keinerlei genauere Informationen.

### Zusammenfassung der Erfindung

Eine Aufgabe der Erfindung besteht darin, einen portablen Applikator zum Einsatz zur pulsierenden Signal-Therapie bereitzustellen, der eine effektivere Stimulation der Kollagenneubildung als die bereits bekannten Vorrichtungen ermöglicht.

Diese Aufgabe wird mit einem portablen Applikator mit den Merkmalen des Anspruchs 1 gelöst.

Der Erfindung liegt der Gedanke zugrunde, dass ein erhöhtes Behandlungsfeld genau an den Stellen vorgesehen wird, an denen es zur Stimulation der Kollagenneubildung benötigt wird. Gemäß diesem Aspekt der vorliegenden Erfindung wird dafür ein portabler Applikator bereitgestellt, der einen Trägerkörper und zumindest zwei Signalerzeugungsanordnungen aufweist, die in oder an dem Trägerkörper angeordnet sind. Die zumindest zwei Signalerzeugungsanordnungen sind in Reihe geschaltet, und jede der zumindest zwei Signalerzeugungsanordnungen umfasst zumindest zwei Kernspulen , die parallel geschaltet sind.

Mittels des erfindungsgemäßen Applikators kann ein erhöhtes Behandlungsfeld genau an den Stellen vorgesehen werden, an denen es zur Stimulation der Kollagenneubildung benötigt wird. Die zu behandelnden Körperteile müssen weder direkt innerhalb einer Spule angeordnet werden, noch ist ein "Gegenpad" erforderlich, d.h. es müssen auch keine zwei Spulen jeweils an gegenüberliegenden Stellen, mit dem zu behandelnden Körperteil dazwischen, vorgesehen werden. Der Applikator der vorliegenden Erfindung ermöglicht somit eine effektivere und gezieltere Anwendung des Magnetfelds auf die entsprechenden Körperteile.

Des Weiteren ist der portable Applikator vorteilhafterweise leicht transportierbar, wie beispielsweise in einem Koffer oder Trolley. Zur entsprechenden Befestigung an den gewünschten Körperteilen weist der Applikator vorzugsweise zusätzliche Befestigungsmittel, wie z.B. Riemen, Gurte, Klebebänder, Klettverschlüsse usw., auf.

Jede Signalerzeugungsanordnung des portablen Applikators umfasst vorzugsweise drei parallel geschaltete Kernspulen. Eine Parallelschaltung von drei Kernspulen ist besonders vorteilhaft, da bei dieser Anzahl eine optimale Feldverteilung zwischen den parallel geschalteten Kernspulen vorliegt, was eine optimale Nutzung des Applikators gewährleistet.

Die Spulen sind mit ihrer Längsachse senkrecht zur Flächenstruktur des Trägerkörpers ausgerichtet, um eine große Eindringtiefe des Magnetfelds in die Haut zu gewährleisten.

Nach einer vorteilhaften Ausführungsform erzeugen die Kernspulen ein pulsierendes magnetisches Feld mit einer Feldstärke von unter 25 Gauß (G), vorzugsweise von 10-20 Gauß. Bei Feldstärken dieser Größenordnung wird gewährleistet, dass das Magnetfeld stark genug ist, um durch die Fettschicht bis zum Knorpel durchzudringen. Auf diese Weise werden durch das angelegte Magnetfeld effektiv Knorpelzellen und (Binde-) Gewebe stimuliert, wobei die Kollagenbildung automatisch mitstimuliert wird.

Vorteilhafterweise sind die Kernspulen in einen Schutzkörper gebettet. Bei einer bevorzugten Ausführungsform wird der Schutzkörper in Form einer Beschichtung direkt auf die Kernspulen aufgebracht. Bei einer anderen bevorzugten Ausführungsform ist der Schutzkörper als eine die Kernspulen abdeckende Kappe ausgebildet.

Vorzugsweise ist der Trägerkörper von einer Ummantelung umgeben, die vorzugsweise aus einem biologisch kompatiblen Material aufgebaut ist. Insbesondere bevorzugt ist die Ummantelung, d.h. der den Körper direkt berührende Teil des portablen Applikators, aus einem hautverträglichen Material ausgebildet. Bei einer besonders bevorzugten Ausführungsform ist die Ummantelung aus Neopren® ausgebildet. Bei einer anderen bevorzugten Ausführungsform ist die Ummantelung aus Latex ausgebildet.

Der Trägerkörper ist vorzugsweise ein elektrisch isolierendes Gel, in das die Signalerzeugungsanordnungen eingebettet sind. Durch die Verwendung eines Gels lässt sich eine optimale Anpassbarkeit der Form des Trägerkörpers an den zu behandelnden Körperteil erzielen.

Gemäß einer bevorzugten Ausführungsform ist der Trägerkörper flächig ausbreitbar. Vorzugsweise weist der Trägerkörper eine dem jeweiligen zu behandelnden Körperteil entsprechende Form auf. Es wird erwägt, die Form des Trägerkörpers so auszugestalten, dass er sich zur Behandlung von Hals oder Dekolleté eignet.

Gemäß einer weiteren bevorzugten Ausführungsform ist der Trägerkörper als Gesichtsmaske ausgebildet. Durch die Ausgestaltung des Applikators als Gesichtsmaske können die faltigen bzw. faltenanfälligen Bereiche des Gesichts einer zu behandelnden Person direkt dem Magnetfeld ausgesetzt werden, wodurch eine besonders effiziente Stimulierung der Kollagenneubildung erreicht wird.

Vorteilhafterweise ist der Trägerkörper mehrteilig ausgebildet. Bei einer bevorzugten Ausführungsform besteht der Trägerkörper aus zwei spiegelsymmetrisch ausgebildeten Teilen. Gemäß einer besonders bevorzugten Ausführungsform sind die mehreren Teile des Trägerkörpers gelenkig und/oder elastisch miteinander verbunden. Dadurch kann ein optimales Aufliegen der Maske gewährleistet werden, auch bei verschiedenen Personen mit jeweils unterschiedlichen Gesichtskonturen. Außerdem wird durch die gelenkige bzw. elastische Verbindung der Trägerkörperteile die Handhabbarkeit verbessert. Beispielsweise kann die Maske gefaltet bzw. zusammengelegt werden, so dass sie beim Transport oder bei der Lagerung weniger Platz einnimmt.

Eine erfindungsgemäße Verwendung ist durch die Merkmale des Anspruchs 11 gekennzeichnet. Dieser Aspekt der vorliegenden Erfindung betrifft insbesondere die Verwendung des oben beschriebenen Applikators zur kosmetischen Hautbehandlung. Eine kosmetische Behandlung umfasst sowohl Wellness- und Anti-Ageing-Behandlungen als auch die kosmetische Behandlung von Wunden und Narben.

Vorteilhafterweise wird bei der Verwendung des Applikators die Neubildung von Kollagen des Typs II, IX, XI und X angeregt. Insbesondere trägt die Neubildung dieser Kollagene dazu bei, das äußere Erscheinungsbild der Haut zu verbessern.

Eine erfindungsgemäße Vorrichtung ist durch die Merkmale des Anspruchs 13 gekennzeichnet. Gemäß diesem weiteren Aspekt der vorliegenden Erfindung wird eine Vorrichtung für die pulsierende Signal-Therapie bereitgestellt, die den oben beschriebenen Applikator und eine Einrichtung zum Zuführen eines Steuersignals (Steuergerät) an die Kernspulen umfasst. Bei einer bevorzugten Ausführungsform sind der Applikator und das Steuergerät als eine kompakte Geräteeinheit vorgesehen, d.h. der Applikator ist mit dem Steuergerät fest verbunden und/oder der Applikator und das Steuergerät sind als eine integrale Einheit in einem gemeinsamen Gehäuse vorgesehen. Zur besseren Transportierbarkeit kann diese Geräteeinheit in einem Koffer oder Trolley angeordnet sein. Bei einer anderen bevorzugten Ausführungsform ist das Steuergerät von dem Applikator lösbar. Auch bei dieser Ausführungsform wird es erwägt, die Vorrichtung zur besseren Transportmöglichkeit in einem Koffer oder Trolley vorzusehen.

Vorzugsweise ist das Steuersignal eine gepulste Gleichspannung mit einer Rate von maximal 30 Pulsen pro Sekunde, besonders bevorzugt 1 bis 12 Pulsen pro Sekunde, insbesondere bevorzugt 5 bis 12 Pulsen pro Sekunde, und eine Rechteckspulsform mit einer abrupt ansteigenden und abrupt abfallenden Spannung. Vorzugsweise beträgt die Spannung 24 V.

Vorteilhafterweise verkleinert das Steuergerät die Impulsbreite über die Betriebsdauer und erhöht die Impulsanzahl über die Betriebsdauer.

Weitere vorteilhafte Ausführungsformen und Verbesserungen der Erfindung sind in den abhängigen Ansprüchen angegeben Die Erfindung umfasst auch

Ausführungsformen, die sich aus einer Kombination von Merkmalen ergeben, die getrennt in den Ansprüchen, den Zeichnungen und der Beschreibung aufgeführt sind.

### Kurze Beschreibung der Zeichnungen

Nachfolgend wird die Erfindung rein beispielhaft anhand der beigefügten Figuren beschrieben, in denen:
Fig. 1 eine schematische Darstellung einer ersten Ausführungsform des erfindungsgemäßen Applikators ist;
Fig. 2 eine schematische Darstellung der Verschaltung der in Fig. 1 dargestellten ersten ist;
Fig. 3 eine schematische Darstellung einer zweiten Ausführungsform des erfindungsgemäßen Applikators ist;
Fig. 4 eine schematische Darstellung einer Modifikation der zweiten Ausführungsform ist;
Fig. 5 eine schematische Darstellung der Verschaltung der in Fig. 4 dargestellten zweiten Ausführungsform des erfindungsgemäßen Applikators ist;
Fig. 6 eine Seitenansicht einer dritten Ausführungsform des erfindungsgemäßen Applikators ist;
Fig. 7 eine Draufsicht des erfindungsgemäßen Applikators der dritten Ausführungsform ist;
Fig. 8 eine schematische Darstellung der Verschaltung der in Fig. 7 dargestellten dritten Ausführungsform ist.

### Detaillierte Beschreibung bevorzugter Ausführungsformen

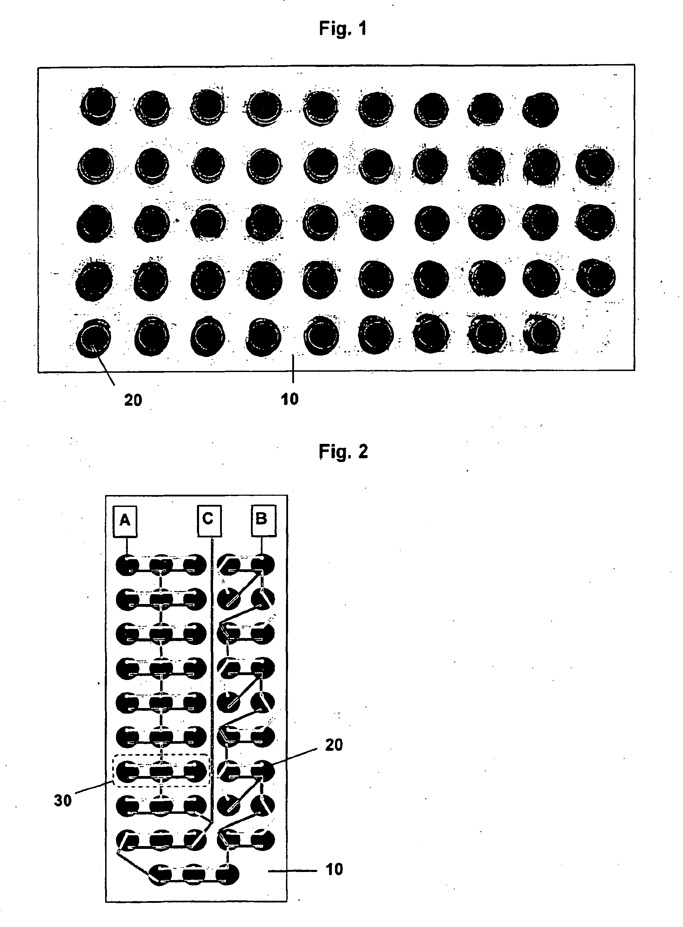

Erfindungsgemäß umfasst ein portabler Applikator einen Trägerkörper 10 und zumindest zwei Signalerzeugungsanordnungen 30, die in oder an dem Trägerkörper 10 angeordnet sind, wie insbesondere in dem Beispiel der Fig. 1 gezeigt. Die zumindest zwei Signalerzeugungsanordnungen 30 sind in Reihe geschaltet, und jede der zumindest zwei Signalerzeugungsanordnungen 30 umfasst zumindest zwei Kernspulen 20, die parallel geschaltet sind. Durch die in Reihe geschalteten zumindest zwei Signalerzeugungsanordnungen 30, die wiederum jeweils zumindest zwei parallel geschaltete Kernspulen 20 aufweisen, kann eine effektivere Stimulation der Kollagenneubildung als mit bereits bekannten Vorrichtungen erreicht werden. Insbesondere kann mittels des erfindungsgemäßen Applikators ein erhöhtes Behandlungsfeld, d.h. ein gezielteres bzw. lokales und intensiveres Magnetfeld, genau an den Stellen vorgesehen werden, an denen es zur Stimulation der Kollagenneubildung benötigt wird.

Vorzugsweise überlagern sich die Magnetfelder der einzelnen Kernspulen 20, wobei die Abstände zwischen den einzelnen Kernspulen an die jeweilige Verwendung angepasst sind. Durch die Überlagerung verstärken sich die einzelnen Magnetfelder, und es wird möglich ein starkes Magnetfeld auf engem Raum, und mit Kernspulen mit geringen Abmessungen, zu erzeugen. Die Erfindung ist allerdings nicht darauf beschränkt, dass sich die Magnetfelder der einzelnen Kernspulen 20 überlagern. Wenn es angemessen ist, können die jeweiligen Kernspulen 20 auch so weit voneinander entfernt angeordnet werden, dass sich die Magnetfelder nicht mehr überlagern. Im Allgemeinen werden die Kernspulen 20 bei einer großflächigen Behandlung der Haut weiter voneinander entfernt angeordnet, während sie bei einer punktuellen Behandlung dichter zusammen angeordnet sind. Eine flächige Behandlung der Haut kann beispielsweise bei der kosmetischen Behandlung von Narben in Betracht gezogen werden. Eine Narbe kann als eine einzelne "Zelle" angesehen werden, zu deren Behandlung sich insbesondere ein "breitflächiges" Magnetfeld, d.h. ein weniger konzentriertes Magnetfeld, eignet. Eine Ausführungsform eines für eine großflächige Hautbehandlung geeigneten Applikators ist die Ausführungsform 1 der vorliegenden Erfindung, die untenstehend noch im Detail beschrieben wird.

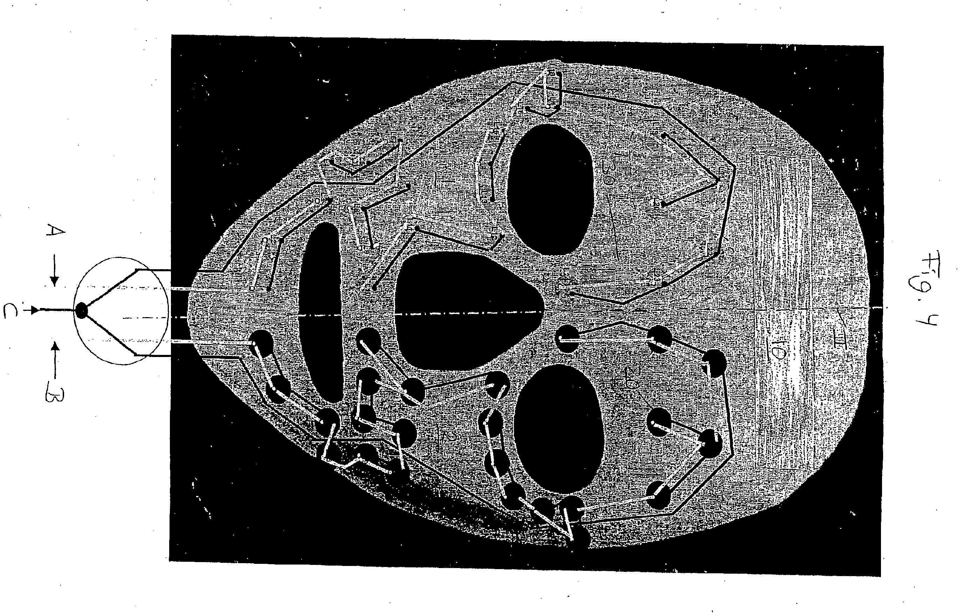

Auf der anderen Seite bietet sich eine Behandlung mit einem punktförmigen Magnetfeld, d.h. einem sehr konzentrierten Magnetfeld, an, wenn sehr viele "Zellen" in einem kleinen Bereich vorhanden sind, wie beispielsweise in dem Bereich unter den Augen. Beispielhafte Ausführungsformen für eine konzentrierte bzw. sehr gezielte und intensive Hautbehandlung sind die erfindungsgemäßen Ausführungsformen 2 und 3, die ebenfalls untenstehend noch ausführlich beschrieben werden. Anhand dieser beispielhaften Ausführungsformen wird verdeutlicht, dass die Kernspulen 20 den jeweiligen Erfordernissen der zu behandelnden Körperstellen entsprechend angeordnet werden können (siehe z.B. Fig. 4, 6 und 7). Beispielsweise sind die Kernspulen 20 in dem Bereich unter den Augen sehr eng beisammen angeordnet, da hier sehr viele Zellen auf kleinem Raum konzentriert sind, wohingegen die Kernspulen 20 im Stirnbereich weiter voneinander beabstandet angeordnet sind, da in diesem Bereich eine weniger hohe Konzentration von Zellen vorliegt.

Der Abstand, in dem die Kernspulen zueinander angeordnet werden, kann somit an den strukturellen Aufbau der zu behandelnden Hautfläche, und damit an die speziellen Behandlungserfordernisse, angepasst werden. Des Weiteren wird der Abstand zwischen den Kernspulen danach gewählt, ob eine Überlagerung der Magnetfelder der jeweiligen Kernspulen gewünscht wird, und wie groß diese Überlagerung sein soll. Wenn eine Überlagerung der Magnetfelder der einzelnen Kernspulen gewünscht wird, müssen die Eigenschaften der verwendeten Kernspulen, wie beispielsweise Abmessungen und Stärke des Magnetfelds, berücksichtigt werden. Vorzugsweise beträgt der Abstand zwischen den einzelnen Kernspulen zwischen 0,5 mal (z.B. im Bereich unterhalb der Augen) und 3 mal (z.B. im Bereich der Stirn) dem Durchmesser einer Kernspulen. Wie der Fachmann verstehen wird, können, je nach Erfordernis und Art der genutzten Spulen, die Abstände zwischen den einzelnen Kernspulen variiert werden.

Im Folgenden werden besonders bevorzugte Ausführungsformen des erfindungsgemäßen Applikators unter Bezugnahme auf die beigefügten Zeichnungen detailliert beschrieben.

### Ausführungsform 1

Fig. 1 ist eine schematische Darstellung einer ersten bevorzugten Ausführungsform des erfindungsgemäßen portablen Applikators. Bei dieser Ausführungsform ist der Applikator flächig ausbreitbar (Flächenapplikator). Insbesondere umfasst der Flächenapplikator einen Trägerkörper 10 sowie eine Vielzahl von Kernspulen 20. Eine Einrichtung zum Zuführen eines Steuersignals zu den Kernspulen ist ebenfalls vorgesehen, wird aber in Fig. 1 nicht gezeigt. Zur entsprechenden Befestigung an dem/den gewünschten Körperteil/en kann der Applikator mit üblichen Befestigungsmitteln, wie z.B. Riemen, Gurte, Klebebänder, Klettverschlüsse usw., versehen sein.

Der in Fig. 1 gezeigte Trägerkörper 10 ist aus einer flexiblen Leiterplatte mit einer rechteckigen Form aufgebaut. Weder das Material des Trägerkörpers 10 noch seine Form sind jedoch auf die in Fig. 1 gezeigte Ausführungsform beschränkt. Vielmehr kann der Trägerkörper 10 aus jedem geeigneten Material ausgebildet sein und jede geeignete Form aufweisen, beispielsweise in Abhängigkeit von dem jeweiligen Körperteil an dem die Anwendung durchzuführen ist. Beispielsweise kann der flächig ausbreitbare Trägerkörper 10 eine rechteckige Form mit abgerundeten Ecken, eine ovale Form, eine runde Form, oder eine nierenförmige Form aufweisen. Die Form kann dem jeweiligen Körperteil, auf das der Trägerkörper 10 aufgelegt wird oder das er umschließt, angepasst werden, um eine optimale Auflage des Trägerkörpers 10 an dem entsprechenden Körperteil zu gewährleisten. Wenn der Trägerkörper aus einem steifen Material ausgebildet ist, kann er, um eine bessere Anpassbarkeit an die Kontur des entsprechenden Körperteils zu ermöglichen, mehrteilig ausgestaltet sein. Diese mehreren Teile können wiederum miteinander gelenkig oder anderweitig, z.B. mit einem Riemen, Klettverschluss etc., verbunden sein. Mögliche Anwendungsbereiche des flächigen Applikators erstrecken sich auch auf den Hals oder das Dekolleté.

Hinsichtlich des Materials des Trägerkörpers 10 werden sowohl steife als auch flexible und/oder elastische Materialien in Betracht gezogen. Neben der in Fig. 1 gezeigten flexiblen Leiterplatte werden auch starre bzw. steife Leiterplatten, flexible bzw. elastische Stoffe/Gewebe sowie flächige Gebilde aus Gummi, und jegliche Materialkombinationen daraus, in Betracht gezogen. Vorzugsweise ist der Trägerkörper 10 aus Neopren® hergestellt. Weitere bevorzugte Materialien für den Trägerkörper 10 umfassen Latex sowie Kunststoffe, insbesondere bevorzugt GVK, PP und/oder PVC. Des Weiteren wird erwägt, den Trägerkörper aus einer Gel-artigen Masse auszubilden. D.h., die Signalerzeugungseinheiten bzw. Behandlungsspulen werden vorzugsweise in eine mit elektrisch isolierendem Gel gefüllten, sich der jeweiligen Körperkontur anpassenden Maske -ähnlich denen zur Augenpflegeeingelassen. In diesem Fall lässt sich der Trägerkörper der jeweiligen Kontur/Form des zu behandelnden Körperteils besonders gut anpassen. Je nach Material des Trägerkörpers 10 sind die Signalerzeugungseinheiten 20 somit entweder an dem Trägerkörper 10 angebracht oder in ihm eingebettet.

Um einen verbesserten Schutz der Signalerzeugungseinheiten 20 gegen äußere Einwirkungen (mechanischer, thermischer und/oder chemischer Art) zu erreichen, können diese zusätzlich in einen Schutzkörper (nicht gezeigt) eingebettet bzw. von ihm umgeben sein. Der Schutzkörper kann sowohl eine als auch mehrere Kernspulen 20 gleichzeitig abdecken, und kann beispielsweise in der Form einer auf die Kernspulen 20 aufsteckbaren Kappe ausgebildet sein. Der Begriff Schutzkörper umfasst jedoch sämtliche Materialien, die sich zum Schutz der Kernspulen 20 eignen, beispielsweise auch Schutzlacke und andere Beschichtungsmaterialien.

Des Weiteren kann auch der Trägerkörper 10 bedeckt bzw. ummantelt sein. Die Ummantelung (nicht gezeigt) des Trägerkörpers 10 kann ein- oder mehrlagig sein. Beispielsweise kann die Ummantelung aus zumindest drei Lagen aufgebaut sein, wobei eine zusätzliche Schicht aus einem sich gut den Konturen des entsprechenden Körperteils anpassenden Material (z.B. Fluid, Gel, Schaum usw.) zwischen einer inneren und einer äußeren Deckschicht angeordnet sein kann. Geeignete Materialien für die Ummantelung des Trägerkörpers 10 umfassen, aber sind nicht beschränkt auf: Stoffe, Gewebe, Neopren®, Gummi, Kunststoffe, wie beispielsweise GVK, PP und/oder PVC, sowie jegliche Materialkombinationen daraus. Da die Ummantelung in direkten Kontakt mit der Haut des Patienten gelangt, sollte das Material der Ummantelung gut hautverträglich sein.

Grundsätzlich gibt es zwei Typen von Spulen: Luftspulen und Kernspulen. Luftspulen haben keinen magnetisch leitenden Kern, der das Magnetfeld verstärkt, und weisen nur sehr kleine Induktivitäten auf. Um praxisgerechte Induktivitäten mit einem geringen Innenwiderstand zu realisieren, sind Spulen mit einem entsprechend großen Drahtquerschnitt nötig, die für die vorliegende Anwendung jedoch ungeeignet sind. Da für die Wellness-/Anti-Ageing-Anwendung relativ kleine Spulen mit einer großen Induktivität wünschenswert sind, werden somit Spulen mit einem Kern aus einem magnetisierbaren Material (z.B. Ferrit oder Eisenpulver-Kern) verwendet, der die Induktivität der Spule vervielfacht.

Die Bauform der Kernspule 20 ist vorzugsweise stehend/radial. Jede Einzelspule kann beispielsweise die folgenden charakteristischen Werte aufweisen: 10.000µH, +/- 10% Toleranz bei einer Testfrequenz von 10 kHz, max. 0,08A DC bei 20°C. Um eine große Eindringtiefe des Magnetfelds bis in die tieferen Hautschichten, d.h. bis zur Faszie, zu gewährleisten, sollten die Spulen mit ihrer Längsachse senkrecht zur Flächenstruktur des Trägerkörpers ausgerichtet sein.

Bei der Einrichtung zum Zuführen eines Steuersignals (nicht gezeigt) zu den Kernspulen 20 handelt es sich vorzugsweise um ein Steuergerät mit den folgenden technischen Daten: Eingang 100-200 VAC, 50-60 Hz, Stromaufnahme max. 2,2A; Ausgang 24VDC, max. -30 Hz. Der äußere Aufbau des Steuergeräts, d.h. sein Gehäuse, kann den jeweiligen Bedürfnissen angepasst ausgestaltet sein. Wenn das Steuergerät portabel ist, kann es beispielsweise zusätzlich einen Tragegriff umfassen. Bei einem Steuergerät, das zum Einbau in eine Geräteeinheit zusammen mit anderen Geräten vorgesehen ist, kann das Gehäuse zusätzliche Einrichtungen zur Befestigung in der Geräteeinheit aufweisen.

Fig. 2 ist eine schematische Darstellung der Verschaltung des in Fig. 1 gezeigten Flächenapplikators. Die Einrichtung zum Zuführen eines Steuersignals wird in Fig. 2 ebenfalls nicht gezeigt. Bei dieser bevorzugten Ausführungsform sind insgesamt achtundvierzig Kernspulen 20 an der Trägerplatte 10 angebracht. Drei Kernspulen 20 bilden wiederum eine Signalerzeugungsanordnung 30, so dass bei dieser Ausführungsform insgesamt sechzehn Signalerzeugungsanordnungen 30 vorgesehen sind. Die drei Kernspulen 20 einer Signalerzeugungsanordnung 30 sind parallel geschaltet, und die Gesamtheit der Signalerzeugungsanordnungen 30 sind in Reihe geschaltet. Obwohl sich diese besonders bevorzugte Ausführungsführungsform auf Signalerzeugungsanordnungen 30 aus drei Kernspulen 20 bezieht, wird der Fachmann verstehen, dass die Signalerzeugungsanordnungen 30 jeweils auch aus einer anderen Anzahl von Kernspulen 20 bestehen können. Beispielsweise sind Ausführungen denkbar, bei denen die Kernspulen 30 aus zwei beziehungsweise vier oder mehr Signalerzeugungseinheiten bestehen.

Bei der in Fig. 2 gezeigten schematischen Darstellung ist der Flächenapplikator in zwei Anschlussseiten unterteilt. Auf jeder der Anschlussseiten ist ein Anschluss A beziehungsweise B angeordnet. Außerdem ist ein gemeinsamer Anschluss C vorgesehen. Obwohl in Fig. 2 so gezeigt, muss nicht auf jeder Anschlussseite die gleiche Anzahl von Signalerzeugungsanordnungen 30 angeordnet sein. Ein Anschlusskabel, zur elektrischen Verbindung des flächig ausbreitbaren Applikators mit dem Steuergerät, kann, durch die vorzugsweise vorhandene Ummantelung des Trägerkörpers, den an jeder beliebigen Stelle vorgesehenen Anschlüssen A, B, C an dem Trägerkörper 10 zugeführt werden.

Erfindungsgemäß erzeugen die Kernspulen 20 ein pulsierendes magnetisches Feld mit einer Feldstärke von unter 25 Gauß, vorzugsweise von 10-20 Gauß. Die Stärke des resultierenden Magnetfelds wird an der dem Körper zuzuwendenden Seite des Applikators gemessen, und ist mit zunehmenden Abstand vom Applikator abnehmend.

### Ausführungsform 2

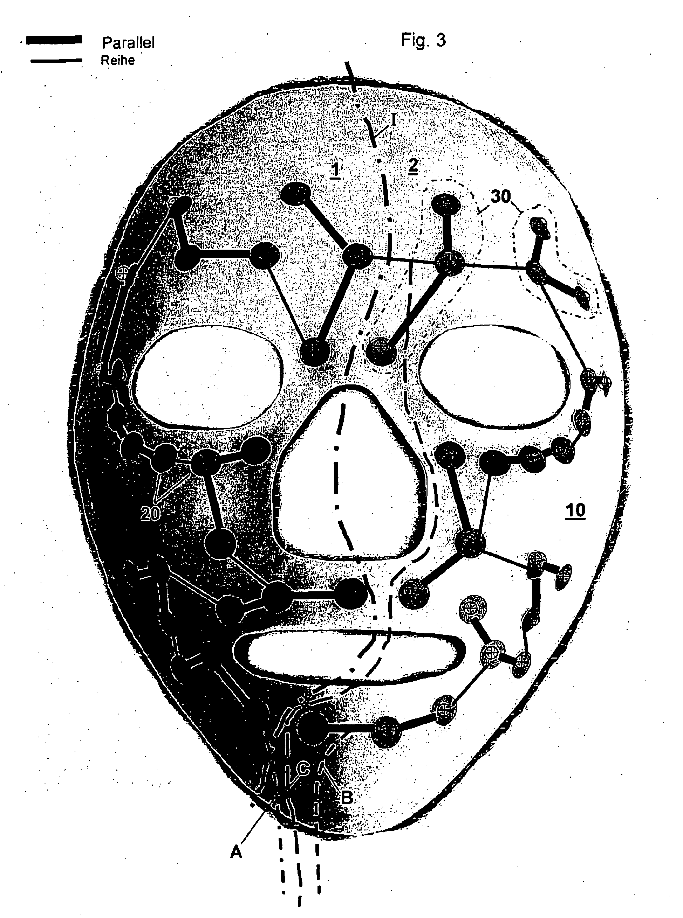

Die in Fig. 3 gezeigte Ausführungsform des erfindungsgemäßen Applikators ist speziell für eine Gesichtsbehandlung vorgesehen. Gleiche und ähnliche Teile wie diejenigen der in den Fig. 1 und 2 gezeigten Ausführungsform 1 werden mit gleichen Bezugszeichen bezeichnet. Außerdem wird lediglich auf die Unterschiede der zwei Ausführungsformen eingegangen. Hinsichtlich der Übereinstimmungen der zwei Ausführungsformen, im Aufbau und in der Funktion, wird auf die obigen Ausführungen zu der Ausführungsform 1 der vorliegenden Erfindung verwiesen.

Bei der in Fig. 3 gezeigten schematischen Darstellung der Ausführungsform 2 des erfindungsgemäßen Applikators (Gesichtsmaske), wird ein Trägerkörper 10 durch eine Trennlinie I in eine rechte Gesichtshälfte 1 und eine linke Gesichtshälfte 2 unterteilt. Vorzugsweise sind auf jeder Seite 1, 2 der Gesichtsmaske acht in Reihe geschaltete Signalerzeugungsanordnungen 30 umfassend jeweils drei parallel geschaltete Kernspulen 20 angeordnet. D.h., auf jeder Seite 1, 2 der Gesichtsmaske sind jeweils 24 Kernspulen 20 angeordnet, womit auf der Gesichtsmaske insgesamt 48 Kernspulen 20 angeordnet sind. Die Kernspulen 20 sind an dem Trägerkörper 10 zur gezielten Stimulation faltiger bzw. faltenanfälliger Regionen des Gesichts vorgesehen. Insbesondere erfolgt die Anordnung der Kernspulen 20 an dem Trägerkörper 10 derart, dass die Kernspulen 20, bei aufgelegter Maske, direkt über den faltigen bzw. faltenanfälligen Bereichen des Gesichts der zu behandelnden Person liegen.

Die beiden Gesichtshälften 1, 2 sind bei der gezeigten Ausführungsform unterschiedlich groß, sie können allerdings in ihrer Gesamtfläche auch gleich groß sein (siehe z.B. Fig. 4). Bei der in Fig. 3 gezeigten Ausführungsform ist die Gesichtsmaske einstückig ausgebildet. Mögliche Materialien für den Trägerkörper sowie einen eventuellen Schutzkörper bzw. eine Ummantelung, wurden bereits unter Bezugnahme auf die erfindungsgemäße Ausführungsform 1 beschrieben.

Vorzugsweise ist die in Fig. 3 beschriebene Gesichtsmaske, sowie die im Folgenden noch zu beschreibenden Ausführungsformen weiterer Gesichtsmasken, aus eingefärbtem glasfaserverstärktem Kunststoff (GVK) hergestellt. Die Herstellung kann in Halbschalentechnik erfolgen, wobei der Hohlraum zwischen den Halbschalen, mit den eingeklebten und verschalteten Kernspulen 20, mit Einkomponenten-Polyurethan-(PU)-Schaum zur Stabilisierung der Form/Halbschalen ausgeschäumt werden kann. Bevorzugte Herstellungsverfahren für die erfindungsgemäßen Masken sind Tiefzieh- oder Spritzgussverfahren.

Die in Fig. 4 gezeigte Gesichtsmaske ist eine Modifikation der in Fig. 3 gezeigten Ausführungsform 2. Wie in Fig. 4 zu sehen ist, unterteilt eine Trennlinie II die Gesichtsmaske in eine rechte Gesichtshälfte 1 und eine linke Gesichtshälfte 2. Vorzugsweise sind beide Gesichtshälften 1, 2 gleich groß, d.h. sie weisen den gleichen Oberflächenbereich auf. Allerdings können die beiden Gesichtshälften 1, 2 auch unterschiedlich groß sein. Auf jeder Gesichtshälfte 1, 2 sind eine Vielzahl von Kernspulen 20 angeordnet. Bei der in Fig. 4 gezeigten Ausführungsform sind die Kernspulen 20 an dem Trägerkörper 10 in Bezug auf die Trennlinie II symmetrisch angeordnet. D.h., auf jeder Gesichtshälfte 1, 2 sind acht Signalerzeugungsanordnungen 30, bestehend jeweils aus drei Kernspulen 20, spiegelsymmetrisch zur Kernspulen Trennlinie II positioniert. Die Anordnung der einzelnen Kernspulen 20 kann jedoch auch individuellen Bedürfnissen angepasst werden, so dass beispielsweise auch eine asymmetrische Anordnung, bzw. eine Anordnung mit einer unterschiedlichen Anzahl von Kernspulen 20 aus jeder Gesichtshälfte 1, 2, denkbar wäre.

Im Unterschied zu der in Fig. 3 gezeigten Ausführungsform, ist die in Fig. 4 gezeigte Maske mehrteilig ausgebildet. Insbesondere besteht die Maske aus zwei Teilen, wobei die Anzahl der Teile nicht auf zwei beschränkt ist. Die zwei Maskenteile (bzw. die beiden Gesichtshälften 1, 2) können gelenkig miteinander verbunden sein, um ein optimales Aufliegen der Maske, bei guter Handhabbarkeit, zu gewährleisten. Der Begriff "gelenkige Verbindung", wie hierin verwendet, umfasst sämtliche Verbindungsarten durch die ein Drehen, Klappen, Falten bzw. Schwenken der mehreren Teile relativ zueinander, entweder in eine oder in mehrere Richtungen, bewirkt werden kann. Zur gelenkigen Verbindung der mehreren Maskenteile werden sämtliche im Stand der Technik bekannte Verbindungsmöglichkeiten, wie z.B. Gelenke, Scharniere, Drehzapfen usw. in Betracht gezogen. Bei einer mehrteiligen Ausgestaltung kann es auch in Betracht gezogen werden, nicht nur gelenkige Verbindungen zwischen den einzelnen Teilen vorzusehen, sondern auch/oder elastische Verbindungen, z.B. Bänder, Riemen, oder andere elastische Mittel oder Materialien im Schnittstellenbereich der beiden Gesichtshälften 1, 2.

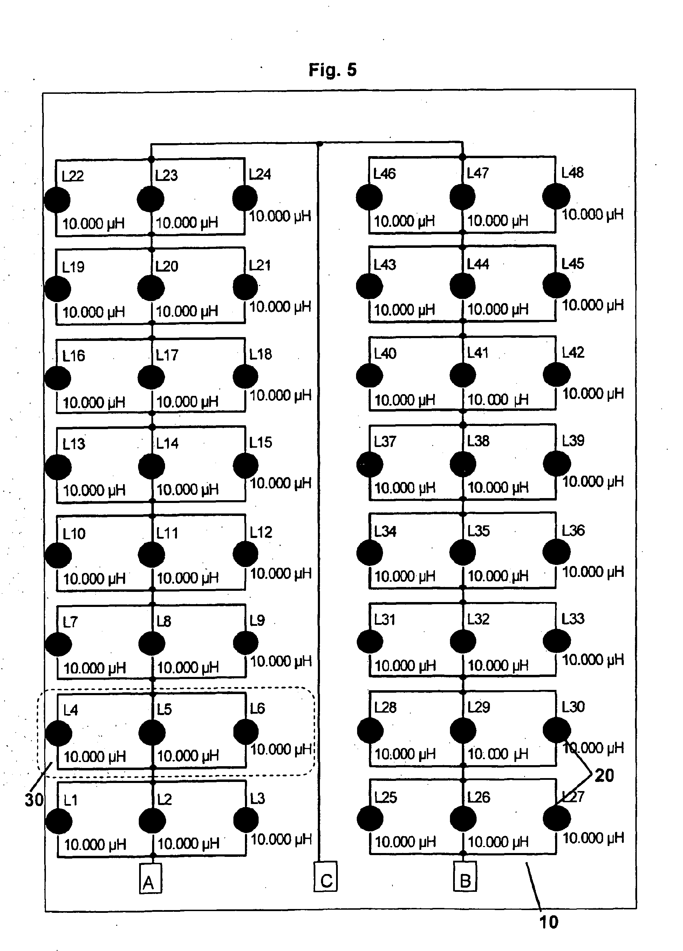

Die in Fig. 3 und 4 gezeigten Gesichtsmasken der erfindungsgemäßen Ausführungsform 2 umfassen jeweils drei Anschlüsse A, B und C, um eine Verbindung mit einem Steuergerät (nicht gezeigt) vorzusehen. Der Anschluss A ist für die Kernspulen 20 der rechten Gesichtshälfte 1 der Gesichtsmaske vorgesehen, der Anschluss B ist für die Kernspulen 20 der linken Gesichtshälfte 2 vorgesehen, und der Anschluss C ist ein gemeinsamer Anschluss. Beispielhaft wird in Fig. 5 schematisch die Verschaltung der in Fig. 4 dargestellten erfindungsgemäßen Ausführungsform der Gesichtsmaske gezeigt.

Wie in Fig. 5 zu sehen ist, umfasst die erfindungsgemäße Gesichtsmaske auf jeder Seite 1, 2 der Maske acht in Reihe geschaltete Signalerzeugungsanordnungen 30 mit jeweils drei parallel geschalteten Kernspulen 20. Bei einer Parallelschaltung von drei Kernspulen 20 ist die Feldverteilung zwischen den Kernspulen 20 optimal. Vorzugsweise weisen außerdem alle drei Kernspulen 20 einer Signalerzeugungsanordnung 30 die gleiche Induktivität auf, so dass jede der drei parallel geschalteten Kernspulen 20 die gleiche Spannung aufweist. Wie in Fig. 5 gezeigt, weisen sämtliche an dem Trägerkörper 10 angeordnete Kernspulen 20 die gleiche Induktivität auf. Somit fließt auf beiden Gesichtshälften 1, 2, aufgrund der gleichmäßigen Anzahl der Kernspulen 20 auf jeder Gesichtshälfte 1, 2, der gleiche Strom.

### Ausführungsform 3

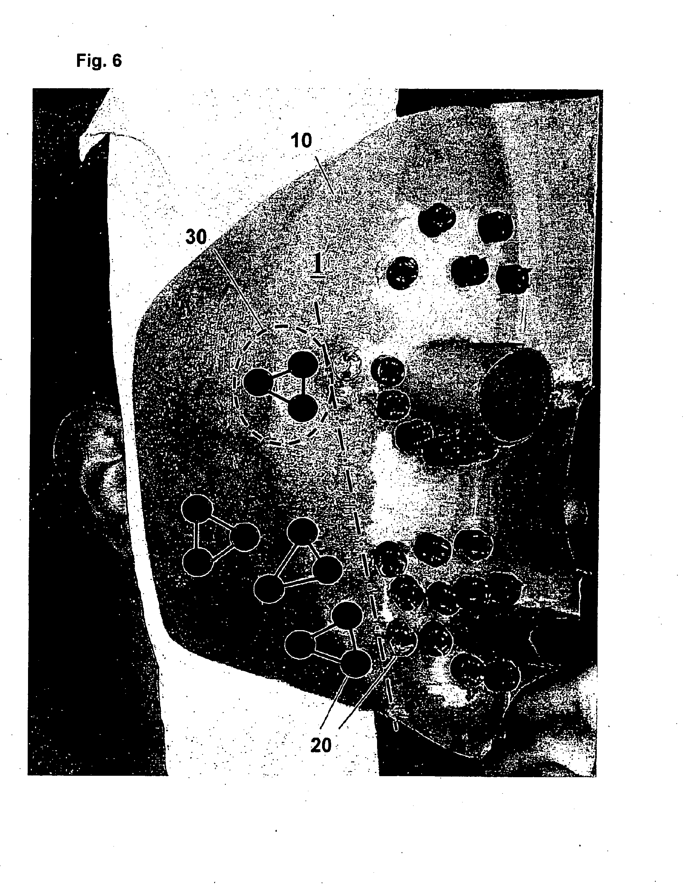

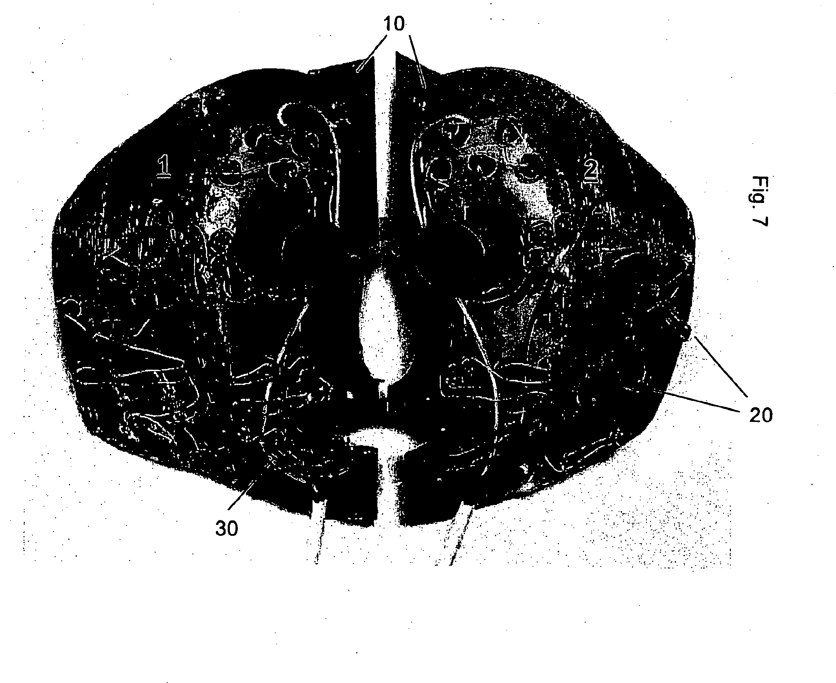

In den Fig. 6 und 7 werden unterschiedliche Ansichten einer Ausführungsform 3 des erfindungsgemäßen Applikators gezeigt. Ähnlich der Ausführungsform 2 ist auch die Ausführungsform 3 speziell für eine Gesichtsbehandlung vorgesehen. Allerdings sieht die Gesichtsmaske der Ausführungsform 3 ein erweitertes Behandlungsfeld vor, wie unten ausführlicher beschrieben wird. Gleiche und ähnliche Teile wie diejenigen der in den Fig. 3, 4 und 5 gezeigten Ausführungsform 2 werden mit gleichen Bezugszeichen bezeichnet. Außerdem wird lediglich auf die Unterschiede der zwei Ausführungsformen eingegangen. Hinsichtlich der Übereinstimmungen der zwei Ausführungsformen, im Aufbau und in der Funktion, wird auf die obigen Ausführungen zu der Ausführungsform 2 der vorliegenden Erfindung verwiesen.

In Fig. 6 wird eine Seitenansicht der Gesichtsmaske gemäß Ausführungsform 3 dargestellt. Insbesondere wird in Fig. 6 lediglich die rechte Gesichtshälfte 1 der Maske, an das Gesicht einer Person gehalten, gezeigt. Fig. 7 hingegen zeigt die Gesichtsmaske gemäß Ausführungsform 3 in Draufsicht, wobei beide Gesichtshälften 1, 2 gezeigt werden. Wie bereits zur Ausführungsform 2 ausgeführt, kann auch die Gesichtsmaske der Ausführungsform 3 ein- oder mehrteilig ausgeführt sein. Falls eine mehrteilige Form der Gesichtsmaske gewählt wird (siehe Fig. 7), können die verschiedenen Einzelteile wiederum gelenkig und/oder elastisch miteinander verbunden werden (nicht gezeigt). Mittels der mehrteiligen Ausgestaltung der Gesichtsmaske, und der gelenkigen und/oder elastischen Verbindung der einzelnen Teile, kann ein optimales Aufliegen der Maske, bei guter Handhabbarkeit, sichergestellt werden.

Des Weiteren wird erwägt die Trägerkörper 10 der rechten und linken Gesichtshälften 1, 2 jeweils mehrteilig und aus unterschiedlichen Materialien auszubilden. Beispielsweise könnte der äußere Abschnitt der jeweiligen Gesichtshälfte 1, 2, auf dem die zusätzlichen jeweils 12 Kernspulen 20 angeordnet sind, aus einem anderen Material ausgebildet werden als der innere Abschnitt der jeweiligen Gesichtshälfte 1, 2, auf dem die übrigen 48 Kernspulen 20 angeordnet sind. Ferner wird in Betracht gezogen, beispielsweise den äußeren oder den inneren Abschnitt aus einem elastischen Material auszubilden, dass dann an dem jeweils anderen Abschnitt befestigt wird. Auf diese Art kann ein optimales Aufliegen der Gesichtsmaske auch ohne eine zusätzliche gelenkige bzw. elastische Verbindung zwischen den einzelnen Teilen sichergestellt werden.

Bei der Ausführungsform 3 sind, zusätzlich zu den bereits in der Ausführungsform 2 verwendeten 48 Kernspulen Kernspulen 20, noch weitere 24 Kernspulen 20 vorgesehen. Die zusätzlichen 20 befinden sich - von der Mitte der Maske aus betrachtet - außerhalb der in den Fig. 6 und 7 gezeigten gestrichelten Linien. Es sind jeweils 4 Signalerzeugungsanordnungen 30 auf jeder Gesichtshälfte 1, 2 an einem Trägerkörper 10 angeordnet. Die Signalerzeugungsanordnungen 30 umfassen jeweils drei Kernspulen 20. Mit dem erfindungsgemäßen Applikator der Ausführungsform 3 wird ein erweitertes Behandlungsfeld geschaffen, so dass auch die Randbereiche des Gesichtsfelds direkt einem Magnetfeld ausgesetzt, und somit effektiv behandelt werden können.

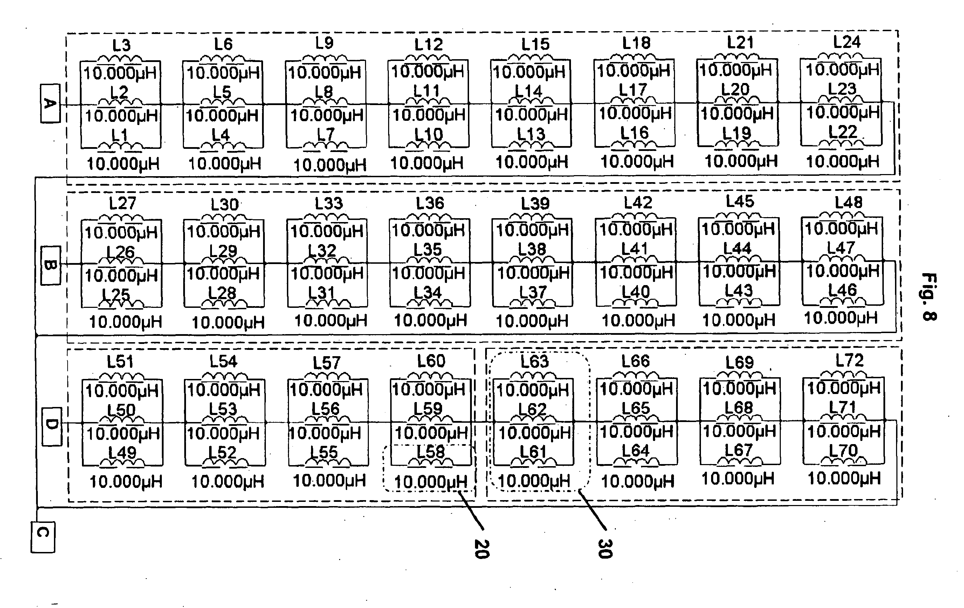

Fig. 8 ist eine schematische Darstellung der Verschaltung der in Fig. 7 dargestellten dritten Ausführungsform. Wie in Fig. 8 gezeigt, ist die Gesichtsmaske mit dem erweiterten Behandlungsfeld in drei Anschlusssektoren unterteilt. Analog zu der Ausführungsform 2 sind auch bei der Ausführungsform 3 Anschlüsse A und B jeweils für einen Teil der Signalerzeugungsanordnungen 30 vorgesehen. Dieser Teil der Signalerzeugungsanordnungen 30 entspricht der Anzahl von Signalerzeugungsanordnungen 30 der Gesichtsmaske der Ausführungsform 2, d.h. die Anschlüsse A und B sind für die ersten insgesamt 48 Kernspulen 20 vorgesehen. Für die weiteren 24 Kernspulen 20, d.h. jeweils 12 Kernspulen 20 auf jeder Gesichtshälfte 1, 2 für das erweiterte Behandlungsfeld, ist ein zusätzlicher Anschluss D vorgesehen. Außerdem ist ein gemeinsamer Anschluss C vorgesehen. Die Verbindung der Anschlüsse mit einem Steuergerät wird nicht gezeigt.

### Verwendung des erfindungsgemäßen Applikators

Rein beispielhaft wird nachfolgend die Verwendung des erfindungsgemäßen Applikators, bzw. der erfindungsgemäßen Vorrichtung, zur kosmetischen Hautbehandlung anhand einer vorteilhaften Ausführungsform beschrieben.

Unter dem Begriff "kosmetische Hautbehandlung", wie hierin verwendet, werden sowohl Wellness- und Anti-Ageing-Behandlungen verstanden, als auch die kosmetische Behandlung von Wunden und Naben. Unter den Begriff "Wellness-Behandlung", wie hierin verwendet, fällt u.a. eine kosmetische Hautbehandlung zur Verbesserung des Hautgefühls und um einen strahlenderen Teint hervorzurufen. Der Begriff "Anti-Ageing-Behandlung", wie hierin verwendet, bezieht sich u.a. auf eine kosmetische Hautbehandlung zur Verringerung der Fältchentiefe und Erhöhung der Straffheit bzw. Festigkeit der Haut. Im Allgemeinen wird durch die kosmetische Anti-Ageing-Behandlung somit ein glatteres und strafferes Erscheinungsbild der Haut erzielt. Des Weiteren werden auch kosmetische Behandlungen von Wunden, Narben sowie bei Hautverfärbungen mit dem erfindungsgemäßen Applikator erwägt. Unter dem Begriff "Hautverfärbungen" werden natürlich auftretende Hautverfärbungen, wie z.B. Altersflecken verstanden, sowie auch traumatisch bedingte Hautverfärbungen, wie beispielsweise aufgrund von Hämatomen oder Ecchymose, oder durch einen chirurgischen oder sonstigen Eingriff bedingt. Die kosmetische Behandlung ist sowohl für jüngere als auch für ältere Menschen geeignet. Insbesondere weisen Personen im Alter für gewöhnlich weniger Muskeln auf, wodurch eine maximale Energie an die zu behandelnden Körperstellen übertragen werden kann.

Zur kosmetischen Hautbehandlung, d.h. zur Stimulierung der Kollagenneubildung, werden elektromagnetische Signale verwendet. Die elektromagnetischen Signale werden durch einen pulsierenden, impulsmodulierten Gleichstrom erzeugt, wobei die Frequenz zwischen 1 und 30 Hz beträgt und die Feldstärke kleiner 25 Gauß, insbesondere zwischen 10 und 20 Gauß ist. Durch die kosmetische Hautbehandlung mit dem erfindgungsgemäßen portablen Applikator soll insbesondere die Neubildung von Kollagen des Typs II, IX, XI und X angeregt werden.

Erfindungsgemäß wird eine Vorrichtung für die pulsierende Signal-Therapie bereitgestellt. Die Vorrichtung umfasst einen Applikator, wie oben im Detail beschrieben, sowie eine Einrichtung zum Zuführen eines Steuersignals (Steuergerät) an die Signalerzeugungseinheiten. Bei einer bevorzugten Ausführungsform sind der Applikator und das Steuergerät als eine kompakte Geräteeinheit vorgesehen. In diesem Fall ist der Applikator mit dem Steuergerät fest verbunden, beispielsweise miteinander verschraubt, und/oder der Applikator und das Steuergerät sind als eine integrale Einheit in einem gemeinsamen Gehäuse vorgesehen. Die Geräteeinheit kann außerdem in einem Koffer oder Trolley vorgesehen sein, um einfacher transportiert werden zu können. Bei einer weiteren bevorzugten Ausführungsform ist das Steuergerät von dem Applikator lösbar. Auch bei dieser Ausführungsform wird es erwägt, die Vorrichtung zur besseren Transportmöglichkeit in einem Koffer oder Trolley vorzusehen.

Während des Betriebs führt das Steuergerät den Kernspulen 20 ein Steuersignal zu, wobei das Steuersignal eine gepulste Gleichspannung mit einer Rate von maximal 30 Pulsen pro Sekunde, vorzugsweise 1 bis 12 Pulsen pro Sekunde, insbesondere bevorzugt 5 bis 12 Pulsen pro Sekunde, und eine Rechteckspulsform mit einer abrupt ansteigenden und abrupt abfallenden Spannung ist. Die Impulsbreite wird über die Betriebsdauer verkleinert, während die Impulsanzahl über die Betriebsdauer erhöht wird. Wie hierin verwendet ist die Impulsbreite die jeweilige Impulsdauer und die Impulsanzahl ist die Anzahl der Impulse pro Zeiteinheit, wobei die Daten jeweils pro Anschlussseite angegeben werden.

Vorzugsweise dauert eine kosmetische Behandlung jeweils ca. eine Stunde pro Sitzung, wobei die Gesamtdauer der Behandlung insgesamt 9 bis 12 Sitzungen beträgt. Bei einer besonders bevorzugten Verwendung erfolgt die Abgabe des Steuersignals über einen Zeitraum von 1h, wobei in den ersten fünf Minuten die höchste Impulsbreite und die niedrigste Impulsanzahl abgegeben werden, von der sechsten bis zur zehnten Minute eine geringere Impulsbreite und eine höhere Impulsanzahl, und von der elften bis zur sechzigsten Minute die geringste Impulsbreite und die höchste Impulszahl abgeben werden. Besonders bevorzugt beträgt die Impulsbreite von der ersten bis zur fünften Minute 40-25ms, insbesondere bevorzugt 35-30ms, von der sechsten bis zur zehnten Minute 30-20ms, insbesondere bevorzugt 26-22ms, und von der elften bis zur sechzigsten Minute 20-10ms, insbesondere 17-13ms. Die Impulsanzahl beträgt von der ersten bis zur fünften Minute 5 bis 8, insbesondere 6, von der sechsten bis zur zehnten Minute 8 bis 10, insbesondere 9, und von der zehnten bis zur sechzigsten Minute 10-12, insbesondere 11. Durch das gegenläufige Verhalten über die Zeit von Impulsbreite und Impulsanzahl durchläuft der sogenannte Tastgrad über die Zeit gesehen ein Maximum; insbesondere tritt das Maximum des Tastgrads zwischen der sechsten und zehnten Minute auf.

Der Tastgrad, auch Tastverhältnis genannt, gibt das Verhältnis der Länge des eingeschalteten Zustands (Impulsdauer) zur Periodendauer bei einem Rechtecksignal an. Der resultierende Wert mit 100% multipliziert ergibt den Tastgrad in Prozent. Bei einer bevorzugten Ausführungsform beträgt der Tastgrad von der ersten bis zur fünften Minute 15-21%, insbesondere 17-20%, von der sechsten bis zur zehnten Minute 20-25%, insbesondere 21-23%, und von der zehnten bis zur sechzigsten Minute 14-20%, insbesondere 15-17%.

Durch das erzeugte Magnetfeld wird die Temperatur der Haut leicht erhöht, ohne jedoch zu einer unangenehmen Erwärmung der Haut zu führen. Beispielsweise sind an der Hautoberfläche gemessene Durchschnittswerte nach zwei Behandlungen mit der Gesichtsmaske der Ausführungsform 2 der vorliegenden Erfindung über eine Dauer von jeweils einer Stunde: Mitte Oberlippe ca. 33°C, Wange links und rechts ca. 32°C, Mitte Stirn ca. 30°C.

Eine Verwendung des oben beschriebenen Applikators zu therapeutischen Zwecken soll beispielhaft beschrieben werden. Eine therapeutische Hautbehandlung kann zum Beispiel die Behandlung von Wunden und Narben zu therapeutischen Zwecken umfassen, wie beispielsweise die Beschleunigung bzw. Förderung der Heilung von Wunden oder die Ausbildung von kleineren bzw. "glatteren" Narben. Die therapeutische Hautbehandlung kann jede Art der Hautbehandlung zu therapeutischen Zwecken umfassen, bei der sich eine Stimulation der Kollagenneubildung positiv auf das Hautbild bzw. den Heilungsprozess auswirkt.

Neben den zuvor beschriebenen. Verwendungen zur kosmetischen oder therapeutischen Hautbehandlung, eignen sich der portable Applikator bzw. die Vorrichtung gemäß der vorliegenden Erfindung auch für eine Reihe von medizinischen Zwecken. Rein beispielhaft sei auf die Behandlung der folgenden Krankheiten verwiesen: Osteoporose, Carpal-Tunnel-Sydrom, Tendinitis, frische Knochenfrakturen und Belastungsfrakturen, aseptische Necrose, Fibromyalgie, Morton's Syndrom, akute Verbrennungen, Epilepsie, Migränekopfschmerzen usw..

Die oben beschriebenen Ausführungsformen der vorliegenden Erfindung sind lediglich beispielhaft und nicht beschränkend. Der Gegenstand der vorliegenden Erfindung wird durch die nachfolgenden Ansprüche definiert.

## Patentansprüche

1. Portabler Applikator für die pulsierende Signal-Therapie, mit:
einem Trägerkörper (10); zumindest zwei Signalerzeugungsanordnungen (30), die in oder an dem Trägerkörper (10) angeordnet sind, wobei die zumindest zwei Signalerzeugungsanordnungen (30) in Reihe geschaltet sind,
und jede der zumindest zwei Signalerzeugungsanordnungen (30) zumindest zwei Kernspulen (20) umfasst, die parallel geschaltet sind, wobei die Kernspulen mit ihrer Längsachse senkrecht zur Flächenstruktur des Trägerkörpers ausgerichtet sind.

2. Portabler Applikator nach Anspruch 1, bei dem jede Signalerzeugungsanordnung (30) drei parallel geschaltete Kernspulen (20) umfasst.

3. Portabler Applikator nach einem der vorhergehenden Ansprüche, bei dem die Kernspulen (20) ein pulsierendes magnetisches Feld mit einer Feldstärke von unter 25G erzeugen.

4. Portabler Applikator nach einem der vorhergehenden Ansprüche, bei dem die Kernspulen (20) in einen Schutzkörper gebettet sind.

5. Portabler Applikator nach einem der vorhergehenden Ansprüche, bei dem der Trägerkörper (10) von einer Ummantelung umgeben ist.

6. Portabler Applikator nach Anspruch 5, bei dem der Trägerkörper (10) ein elektrisch isolierendes Gel ist, in das die Signalerzeugungsanordnungen (30) eingebettet sind.

7. Portabler Applikator nach einem der vorhergehenden Ansprüche, bei dem der Trägerkörper (10) flächig ausbreitbar ist.

8. Portabler Appliaktor nach einem der Ansprüche 1 bis 6, bei dem der Trägerkörper (10) als Gesichtsmaske ausgebildet ist.

9. Portabler Applikator nach Anspruch 7 oder 8, bei dem der Trägerkörper (10) mehrteilig ausgebildet ist.

10. Portabler Applikator nach Anspruch 9, bei dem die mehreren Teile des Trägerkörpers (10) gelenkig und/oder elastisch miteinander verbunden sind.

11. Verwendung eines Applikators nach einem der Ansprüche 1 bis 10 zur kosmetischen Hautbehandlung.

12. Verwendung nach Anspruch 11, wobei die Neubildung von Kollagen des Typs II, IX, XI und X angeregt wird.

13. Vorrichtung für die pulsierende Signal-Therapie, mit einem Applikator nach einem der Ansprüche 1 bis 10 und einer Einrichtung zum Zuführen eines Steuersignals an die Kernspulen (20).

14. Vorrichtung nach Anspruch 13, wobei das Steuersignal eine gepulste Gleichspannung mit einer Rate von maximal 30 Pulsen pro Sekunde, vorzugsweise 1 bis 12 Pulsen pro Sekunde, insbesondere bevorzugt 5 bis 12 Pulsen pro Sekunde, und eine Rechteckspulsform mit einer abrupt ansteigenden und abrupt abfallenden Spannung ist.

15. Vorrichtung nach Anspruch 14, wobei das Steuergerät die Impulsbreite über die Betriebsdauer verkleinert und die Impulsanzahl über die Betriebsdauer erhöht.

16. Vorrichtung nach einem der Ansprüche 13 und 14; bei der das Steuergerät von dem Applikator lösbar ist.

## Claims

1. Portable applicator for pulsed signal therapy, with:
a support body (10); at least two signal-generating arrangements (30), which are positioned in or
on the support body (10), whereby the at least two signal-generating arrangements (30) are connected in series, and each of the at least two signal-generating arrangements (30) includes at least two cored coils which are connected in parallel, wherein the cored coils are aligned perpendicularly with their longitudinal axis to the surface structure of the supporting body.

2. Portable applicator according to claim 1, where each signal-generating arrangement (30) includes three cored coils (20) connected in parallel.

3. Portable applicator according to any of the preceding claims, where the cored coils (20) produce a pulsed magnetic field with a field-strength of less than 25 G.

4. Portable applicator according to any of the preceding claims, where the cored coils (20) are imbedded in a protective body.

5. Portable applicator according to any of the preceding claims, where the support body (10) is surrounded by a coating.

6. Portable applicator according to claim 5, where the support body (10) is an insulating gel, in which the signal-generating arrangements (30) are imbedded.

7. Portable applicator according to any of the preceding claims, where the support body (10) is malleable.

8. Portable applicator according to any of the claims 1 to 6, where the support body (10) is built as a facial mask.

9. Portable applicator according to claim 7 or 8, where the support body (10) is built in several parts.

10. Portable applicator according to claim 9, where the several parts of the support body (10) are connected with each other in an articulated and/or elastic manner.

11. Use of an applicator according to any of the claims 1 to 10 for the cosmetic treatment of skin.

12. Use according to claim 11, whereby the regeneration of collagen types II, IX, XI and X is stimulated.

13. Device for pulsed signal therapy, with an applicator according to any of the claims 1 to 10 and a device for the feed of the control signal to the cored coils (20).

14. Device according to claim 13, whereby the control signal is a pulsed direct current voltage with maximal rate of 30 pulses per second, preferably 1 to 12 pulses per second, and more preferably 5 to 12 pulses per second, and is a rectangular pulse form with an abrupt increase and an abrupt plunge in voltage.

15. Device according to claim 14, whereby the control module reduces the pulse broadness throughout the operating time and increases the pulse amount throughout the operating time.

16. Device according to any of the claims 13 to 14, where the control module is detachable from the applicator.

## Revendications

1. Applicateur portable pour thérapie par signaux pulsés, comprenant :
un corps support (10) ; au moins deux dispositifs de génération de signaux (30) qui sont disposés dans ou au niveau du corps support (10), où les dispositifs de génération de signaux (30), au moins au nombre de deux,
sont montés en série, et chacun des dispositifs de génération de signaux (30), au moins au nombre de deux, comporte au moins deux bobines à noyau (20) qui sont montées en parallèle, les bobines à noyau étant orientées par leur axe longitudinal perpendiculairement à la structure de surface du corps support.

2. Applicateur portable selon la revendication 1, dans lequel chaque dispositif de génération de signaux (30) comporte trois bobines à noyau (20) montées en parallèle.

3. Applicateur portable selon l'une des revendications précédentes, dans lequel les bobines à noyau (20) génèrent un champ magnétique pulsé présentant une intensité de champ inférieure à 25 G.

4. Applicateur portable selon l'une des revendications précédentes, dans lequel les bobines à noyau (20) sont insérées dans un corps protecteur.

5. Applicateur portable selon l'une des revendications précédentes, dans lequel le corps support (10) est entouré d'une enveloppe.

6. Applicateur portable selon la revendication 5, dans lequel le corps support (10) est un gel électriquement isolant dans lequel sont insérés les dispositifs de génération de signaux (30).

7. Applicateur portable selon l'une des revendications précédentes, dans lequel le corps support (10) peut s'étaler en nappe.

8. Applicateur portable selon l'une des revendications 1 à 6, dans lequel le corps support (10) est exécuté en tant que masque pour visage.

9. Applicateur portable selon la revendication 7 ou 8, dans lequel le corps support (10) est exécuté en plusieurs pièces.

10. Applicateur portable selon la revendication 9, dans lequel les différentes pièces du corps support (10) sont reliées entre elles par articulation et/ou de façon élastique.

11. Utilisation d'un applicateur selon l'une des revendications 1 à 10 pour un traitement cosmétique de la peau.

12. Utilisation selon la revendication 11, dans laquelle on stimule la néoformation de collagène de type II, IX, XI et X.

13. Dispositif de thérapie par signaux pulsés, comportant un applicateur selon l'une des revendications 1 à 10 et un dispositif permettant d'amener un signal de commande aux bobines à noyau (20).

14. Dispositif selon la revendication 13, dans lequel le signal de commande est une tension continue pulsée selon une fréquence de 30 pulsations par seconde au maximum, de préférence de 1 à 12 pulsations par seconde, de façon particulièrement préférée de 5 à 12 pulsations par seconde, et une forme de pulsation rectangulaire avec une tension qui augmente de façon abrupte et chute de façon abrupte.

15. Dispositif selon la revendication 14, dans lequel l'appareil de commande réduit la largeur d'impulsion sur la durée de fonctionnement et augmente le nombre d'impulsions sur la durée de fonctionnement.

16. Dispositif selon l'une des revendications 13 et 14, dans lequel l'appareil de commande peut être détaché de l'applicateur.
